# EUROPEAN PATENT APPLICATION

(11) **EP 3 951 787 A1**
(43) Date of publication of application: **09.02.2022**
(21) Application number: 20189070.4
(22) Date of filing: 02.08.2020
(51) Int. Cl.: G16H 20/17

(54) **METHOD FOR DISPLAYING A MEDICAL VALUE**

(71) Applicant: Roche Diabetes Care GmbH, 68305 Mannheim (DE); F. Hoffmann-La Roche AG, 4070 Basel (CH)
(72) Inventor: MORENO SANCHEZ, Miguel Angel, 1010 Wien (AT)
(74) Representative: Meinel, Anne Julia

(57) **Abstract**

The present invention relates to a computer-implemented method, a mobile device, and a computer readable storage medium, for displaying a medical value such as, e.g., a glucose value, on a screen of a mobile device.

## Description

### FIELD OF THE INVENTION

The present invention relates to a computer-implemented method, a mobile device, and a computer readable storage medium, for displaying a medical value such as, e.g., a glucose value, on a screen of a mobile device.

### BACKGROUND OF THE INVENTION

Diabetes mellitus, often referred to as diabetes, is a chronic condition that results from defects in the body's ability to produce and/or use insulin. There are three main types of diabetes. Type 1 diabetes usually strikes children and young adults, and may be autoimmune, genetic, and/or environmental. Type 2 diabetes accounts for 90-95% of diabetes cases and is linked to obesity and physical inactivity. Gestational diabetes is a form of glucose intolerance diagnosed during pregnancy and usually resolves spontaneously after delivery.

High blood sugar levels may cause an array of physiological side effects, e.g., associated with the deterioration of small blood vessels. These side effects may include, for example, kidney failure, skin ulcers, bleeding into the vitreous of the eye, etc. Low blood sugar levels may be associated with a risk for headache, seizure, loss of consciousness, and coma.

Diabetes is managed primarily by controlling the level of glucose in the bloodstream. This level is dynamic and complex, and is affected by multiple factors including the amount and type of food consumed, and the amount of insulin (which mediates transport of glucose across cell membranes) in the blood. Blood glucose levels are also sensitive to exercise, sleep, stress, smoking, travel, illness, menses, and other psychological and lifestyle factors unique to individual patients.

A number of systems have been developed for measuring glucose levels with a device located in, on or near the body of a user. These devices typically include one or more glucose sensors and communication circuitry for wirelessly transmitting the data or other information from the sensor to an electrical device for monitoring or displaying the data, optionally via intermediate electrical device(s).

A common application for devices such as continuous glucose monitoring devices is the automatic monitoring of glucose in bodily fluid such as in in interstitial fluid (ISF) or blood of the wearer. Such analyte monitoring devices have particular value in the management of diabetes. For example, for continuous glucose monitoring (CGM) a body sensor inserted under the skin or into the body may be used to check glucose levels. The sensor stays in place for several days to weeks or months and then must be replaced. A transmitter may send information about glucose levels (e.g., via wireless data transmission) from the sensor to a device for monitoring or displaying.

Knowing the current analyte level and how it may change over time is important for determining the appropriate treatment for managing the diabetes condition.

Initially, (continuous) glucose monitoring device wirelessly transmitted data relating to glucose levels to a dedicated medical device designed specifically to display glucose levels, and other information for a user (patient). However, with the increasing popularity of smart phones and software applications (apps) executing on smart phones, more and more users prefer to avoid having to carry a dedicated medical device. Instead, they prefer to monitor their glucose levels using a software app executing on their mobile computing device, such as a smart phone, tablet or wearable device like a smartwatch or smartglasses.

US10596318B2 discloses a CGM app which allows patient users to view and dismiss glucose notifications in the manner most convenient for them, e.g., from their smartphone device's lock screen or their smartwatch. It is also disclosed that the CGM app can manage presentation of the data using a prominent tool of the operating system (e.g., Today Widget of Apple iOS) of the patient user's smartphone or wearable device (e.g., Apple Watch), enabling the user to glanceably view glucose and insulin data and make instant decisions for their treatment.

AU2015335913A1 describes that one or more of the current glucose level and the rate of change may be displayed on a locked screen when the device is locked.

US9585563B2 describes sending a notification message to a remote monitor based on received sensor data.

US10406287B2 describes that a smart alert may be provided on the lock screen of a smart phone within the context of a low glucose alert. When a user sees a notification on their smart phone, they may unlock their phone and see a "pop over" notification of the alert.

US10328204B2 discloses a CGM receiver or CGM smart phone application which can display an indicia of the appertaining user when displaying the user's CGM data.

US20170220750A1 discloses that notifications may be provided on a phone lock screen as well as on a smart watch, presenting different button options to users to allow feedback to the app without having to enter the app.

US20150289821A1 discloses a number of types of features employable on mobile device user interfaces for indicating actionable alerts based on GUI values. The user interfaces in particular may show a glycemic urgency index and glycemic or glucose information. The indications of GUI values may be made in a number of ways, such as the use of visualizations or representations, such as colors or elements such as icons..

Unrelated to medical values, US8904479B1 discloses a display showing a status bar along its top edge. As an example, such a status bar shows, e.g., the presence or absence of WiFi or Bluetooth, and additionally, the status bar also shows graphical elements for notifications of voice mails since the screen was last operating, missed calls, text messages and received e-mail messages.

While the prior art might describe displaying glucose information on a screen of a mobile device, the operating system of the mobile device might not allow the manufacturer of a (diabetes) application to display medical data on the screen of the mobile device fully at his discretion. In particular, in the locked state of the mobile device and/or outside of the diabetes application there are limitations which hinder discretely and quickly providing up to date glucose information to the user.

It is an object of the present disclosure to provide improved technology for displaying a medical value, such as a glucose value, to the user on a screen of a mobile device and thereby providing information for determining the appropriate treatment for managing his medical condition (status), such as his diabetes condition.

### SUMMARY OF THE INVENTION

This task is solved by the method provided herein. Particular embodiments, which might be realized in an isolated fashion or in any arbitrary combination, are listed in the dependent claims.

In one aspect the present invention relates to a computer implemented method of displaying a medical value on a screen of a mobile device comprising a medical application, the method comprising the following steps:
- receiving on the mobile device a first medical value and optionally a timestamp associated with the first medical value indicating the time of measurement of the first medical value
- converting the first medical value to a first text if the first medical value is not directly received as text
- creating first style information for drawing
- creating a first drawing area
- drawing the first text on the first drawing area using the first style information to create an image of the first medical value
- creating a first medical value icon from the image of the first medical value
- displaying the first medical value icon on the screen of the mobile device, wherein the screen is a home screen, a lock screen or another screen outside of the medical application,
optionally wherein the step of displaying of the first medical value icon, and none, one or more of the steps of converting the first medical value to a first text, creating the first style information, creating the first drawing area, drawing the first text on the first drawing area, and creating the first medical value icon are only conducted if the difference between the time of measurement of the first medical value and the current time does not exceed a predefined time threshold.

In another aspect the invention relates to a mobile device having at least one display, the mobile device being configured for performing the method as described above.

In another aspect, the invention relates to a computer program comprising instructions which, when the program is executed by a mobile device having a display, cause the mobile device to carry out the method as described above.

In another aspect, the invention relates to a computer-readable storage medium, specifically a non-transitory storage medium, comprising instructions which, when executed by a mobile device having a display, cause the mobile device to carry out the method as described above.

### DETAILED DESCRIPTION OF THE INVENTION

As used in the following, the terms "have", "comprise" or "include" or any arbitrary grammatical variations thereof are used in a non-exclusive way. Thus, these terms may both refer to a situation in which, besides the feature introduced by these terms, no further features are present in the entity described in this context and to a situation in which one or more further features are present. As an example, the expressions "A has B", "A comprises B" and "A includes B" may both refer to a situation in which, besides B, no other element is present in A (i.e. a situation in which A solely and exclusively consists of B) and to a situation in which, besides B, one or more further elements are present in entity A, such as element C, elements C and D or even further elements.

Further, it shall be noted that the terms "at least one", "one or more" or similar expressions indicating that a feature or element may be present once or more than once typically will be used only once when introducing the respective feature or element. In the following, in most cases, when referring to the respective feature or element, the expressions "at least one" or "one or more" will not be repeated, non-withstanding the fact that the respective feature or element may be present once or more than once.

Further, as used in the following, the terms "preferably", "more preferably", "particularly", "more particularly", "specifically", "more specifically" or similar terms are used in conjunction with optional features, without restricting alternative possibilities. Thus, features introduced by these terms are optional features and are not intended to restrict the scope of the claims in any way. The invention may, as the skilled person will recognize, be performed by using alternative features. Similarly, features introduced by "in an embodiment of the invention" or similar expressions are intended to be optional features, without any restriction regarding alternative embodiments of the invention, without any restrictions regarding the scope of the invention and without any restriction regarding the possibility of combining the features introduced in such way with other optional or non-optional features of the invention.

As set out above, in a first embodiment the invention relates to a computer implemented method of displaying a medical value on a screen of a mobile device comprising a medical application, the method comprising the following steps:
- receiving on the mobile device a first medical value and optionally a timestamp associated with the first medical value indicating the time of measurement of the first medical value
- converting the first medical value to a first text if the first medical value is not directly received as text
- creating first style information for drawing
- creating a first drawing area
- drawing the first text on the first drawing area using the first style information to create an image of the first medical value
- creating a first medical value icon from the image of the first medical value
- displaying the first medical value icon on the screen of the mobile device, wherein the screen is a home screen, a lock screen or another screen outside of the medical application,
optionally wherein the step of displaying of the first medical value icon, and none, one or more of the steps of converting the first medical value to a first text, creating the first style information, creating the first drawing area, drawing the first text on the first drawing area, and creating the first medical value icon are only conducted if the difference between the time of measurement of the first medical value and the current time does not exceed a predefined time threshold. Thereby (up to date) information is provided to the patient (the user of the mobile device) about his medical condition for determining the appropriate treatment.

The term "mobile device" may refer to a mobile electronics device, more specifically to a mobile communication device such as a cell phone or smart phone. Additionally or alternatively, the mobile device may also refer to a smart watch, a tablet computer or another type of portable computer. The mobile device comprises a display (e.g. a touch sensitive display) for displaying a medical value to a patient on a screen. The patient may be also termed a user of the mobile device. The mobile device may also comprise an operating system, e.g. Android. The mobile device further comprises a medical application (app). The medical app is installed on the mobile device.

The medical value(s) may particularly be glucose value(s), particularly continuous glucose monitoring value(s). A medical value may particularly indicate the concentration of an analyte in a bodily fluid of a patient. In one example, the analyte is glucose. The term medical value may thus particularly refer to a glucose value, particularly the blood glucose concentration of a patient. A glucose value may alternatively indicate the glucose concentration in interstitial fluid, urine, saliva or another type of body fluid of a patient. If there are more than one medical values received by the mobile device in the present invention (i.e. if a second medical value and/or a further medical value is received), they are of the same type of medical value as the first medical value and are determined for the same patient as the first medical value. For example, the first medical value, the second medical value and the further medical value are, e.g., all glucose values. The medical value(s) may alternatively be triglyceride value(s), lactate value(s), or cholesterol value(s) or ketone value(s).

The medical values(s) can be received on the mobile device wirelessly from, e.g., a medical sensor such as a continuous glucose monitoring sensor, a separate electronic device or the cloud via a receiving unit. The receiving unit may comprise an interface such as a Bluetooth interface in order to receive and transmit data. The timestamp(s) associated with the medical value(s), respectively, may be received the same way as the medical value(s). The timestamp associated with a medical value indicates when the respective medical value was measured.

The medical value(s) may be received on the mobile device in any format (data type), e.g. directly in text format. As an alternative, the medical value is received in a format other than text e.g. as a numeric value such as an integer. If the medical value is not directly received on the mobile phone as text, it is then converted to text. The conversion may be done directly to text or the conversion may be to an intermediate format first (e.g. (double-precision) floating-point format) and then to text.

The term of "creating first style information for drawing" refers to defining properties for the image to be created. Style information may particularly include information on at least one of color, text size and text alignment. A paint object holding the style information about how to draw may thus be created. For creating the first style information for drawing the style information may be retrieved from a memory of the mobile device.

The term of "creating a first drawing area" refers to defining a drawing area comprising a certain width and height and/or a grid of pixels. Creating a first drawing area may thus comprise creating a bitmap to hold the pixels where a canvas will draw into. Creating the first drawing area may further comprises retrieving information on the width and height of the drawing area to be created or on the grid of pixels from the memory of the mobile phone.

Drawing commands may be generated for drawing the first text on the first drawing area. The term of "drawing the first text on the first drawing area" may thus comprises applying drawing commands which use the first style information to create an image of the first medical value. The method of the present invention may thus comprise creating a canvas passing the bitmap and drawing the first text into the bitmap using the paint.

The term of an icon as used herein is defined as a symbol or graphical representation to be displayed on the screen of a mobile device. In the present invention, the icon is particularly a notification icon.

The term of "creating a first medical value icon from the image of the first medical value" may thus refers to the creation of a symbol or graphical representation of the medical value from the created image of the first medical value in a defined file format for display on the home screen, lock screen or another screen outside of the medical application. The icon is accepted by the operating system of the mobile device for display on a home screen, a lock screen or another screen outside of the medical application. The display of text might not be accepted on at least parts of such screens. However, (notification) icons are accepted for display in these parts of the screens.

A medical application (also termed medical app) may be defined as a software program (e.g. downloadable from an App store) that runs on the mobile device. A medical app is particularly a software program by a manufacturer different from the manufacturer of the mobile device and the operating system of the mobile device. The medical app may particularly be configured to carry out one or more of the receiving, converting, retrieving, creating, drawings, and determining steps of the method of the present invention. The medical app may further be configured for causing the operating system of the mobile device to display the medical value icon or the predefined icon, on the home screen, the lock screen or another screen outside of the medical application.

The home screen is a screen allowing the patient to select which one of a plurality of applications (programs) installed on the mobile device is to be executed in the foreground. When an application is executed in the foreground, a screen of this application is displayed. When an application is not executed in the foreground, a screen outside of the application is displayed.

The lock screen is a screen shown when the mobile device is in a locked state. Patient authentication may be required from the patient in order to unlock the mobile device. In the locked state, the patient may be unable to interact with applications running on the phone. When the phone is in an unlocked state, a patient may be able to interact with applications running on the mobile device.

The home screen and the lock screen are exemplary screens outside of the medical application. Screens inside of an application are screens of the medical application when it is running in the foreground.

In one embodiment, the step of displaying of the first medical value icon, and none, one or more of the steps of converting the first medical value to a first text, creating the first style information, creating the first drawing area, drawing the first text on the first drawing area, and creating the first medical value icon are only conducted if the difference between the time of measurement of the first medical value and the current time does not exceed a predefined time threshold. The method of the present invention may thus comprises determining, using the timestamp associated with the first medical value, whether the difference between the time of measurement of the first medical value and the current time exceeds a predefined time threshold. Checking for an exceedance of the predefined time threshold and, in the affirmative, refraining from displaying the first medical value icon ensures that only recent information is provided to the user of the mobile device regarding his medical status. Outdated information may lead, e.g., to a wrong medical treatment which can cause serious complications for the patient.

The method of the present invention may further comprise the following steps
- receiving on the mobile device a second medical value after having received the first medical value and optionally a timestamp associated with the second medical value indicating the time of measurement of the second medical value which is after the time of measurement of the first medical value
- converting the second medical value to a second text if the second medical value is not directly received as text
- retrieving the first style information or creating second style information for drawing
- creating a second drawing area
- drawing the second text on the second drawing area using the first style information or the second style information to create an image of the second medical value
- creating a second medical value icon from the image of the second medical value
- replacing the display of the first medical value icon on the screen of the mobile device by displaying the second medical value icon on the screen of the mobile device instead,
optionally wherein the step of displaying the second medical value icon, and none, one or more of the steps of converting the second medical value to second text, retrieving the first style information or creating the second style information, creating the second drawing area, drawing the second text on the second drawing area, and creating the second medical value icon are only conducted if the difference between the time of measurement of the second medical value and the current time does not exceed the predefined time threshold.

The second medical value is determined for the same patient as the first medical value and it is of the same type of medical values as the first medical value. The first and second medical values thus both may be continuous glucose monitoring values. The second medical value is received on the mobile device after the first medical value and the time of measurement of the second medical value is after the time of measurement of the first medical value. The display of the first medical value icon on the screen of the mobile device can be replaced by displaying the second medical value icon on the screen of the mobile device instead, wherein the screen is a home screen, a lock screen or another screen outside of the medical application. Thereby, the patient may always be provided with most up to date information. For drawing the second text on the second drawing area the retrieved first style information may be reused or second style information are created and used to create an image of the second medical value. The former process may make the method even more efficient. The first and/or second style information for drawing may be based on a first set of style data stored in the memory of the mobile device. Also, the creation of the first and/or second drawing area may be based on a first set of drawing area data stored in a memory of the mobile device.

In one embodiment, the step of displaying of the second medical value icon, and none, one or more of the steps of converting the second medical value to second text, retrieving the first style information or creating the second style information, creating the second drawing area, drawing the second text on the second drawing area, and creating the second medical value icon are only conducted if the difference between the time of measurement of the second medical value and the current time does not exceed the predefined time threshold. The method of the present invention may thus comprises determining, using the timestamp associated with the second medical value, whether the difference between the time of measurement of the second medical value and the current time exceeds a predefined time threshold. Checking for an exceedance of the predefined time threshold and, in the affirmative, refraining from displaying the second medical value icon ensures that only recent information is provided to the patient of the mobile device regarding his medical status. Outdated information may lead, e.g., to a wrong medical treatment which can cause serious complications for the patient.

The method of the present invention may further comprise
- determining or re-determining whether the difference between the measurement time of the first, or if a second medical value was received, the measurement time of the second medical value and the current time exceeds the predefined time threshold, and
if the difference between the time of measurement of the first, or if a second medical value was received, the time of measurement of the second medical value and the current time exceeds the predefined time threshold, replacing the display of the currently displayed medical value icon on the display of the mobile device by displaying a predefined icon stored in the memory of the mobile device. The predefined icon may also be termed unknown current medical status icon herein.

The predefined icon as an example includes a graphical representation of a question mark. In contrast to the medical value icons, the predefined icon is not newly created ("on the fly") each time a new medical value is received, but the same predefined icon can be used each time it is determined that the currently displayed medical value gets too old and no newer medical value has been received. Thereby the patient gets alerted that no up to date medical value has been received on the mobile device. The patient might thus be careful about his (unknown) state of his medical condition. This might also be, e.g., a trigger for the patient to check the wireless connection between the mobile device and the sensor or other external device or cloud connection via which the medical value might be received by the medical device.

The method of the preceding claim may further comprise
- if the difference between the time of measurement of the first, or if a second medical value was received, the time of measurement of the second medical value and the current time does not exceed the predefined time threshold continuing the displaying of the currently displayed medical value icon.

Also thereby, it may, e.g., be checked every few minutes that the currently displayed medical value icon is still up to date.

The method of the present invention may further comprise
- receiving on the medical device a further medical value and a timestamp associated with the further medical value indicating the time of measurement of the further medical value
- determining whether the difference between the time of measurement of the further medical and the current time does not exceed the predefined time threshold,
- if the time threshold is not exceeded
- converting the further medical value to a further text if the further medical value is not directly received as text
- retrieving the first style information or creating further style information for drawing
- creating a further drawing area
- drawing the further text on the further drawing area using the first style information or the further style information to create an image of the further medical value
- creating a further medical value icon from the image of the further medical value
- replacing the display of the currently displayed medical value icon on the display of the mobile device by displaying the further medical value icon on the display of the mobile device instead or replacing the display of the currently displayed predefined icon on the display of the mobile device instead.

The further medical value is determined for the same patient as the previously received medical value and it is of the same type of medical values as the previously received medical value. The further medical value thus may also be a continuous glucose monitoring value.

The medical value(s) as referred herein may thus (all) be glucose value(s), particularly continuous glucose monitoring value(s).

The predefined threshold may be less than 30 min. As an example, the predefined threshold may be between 1 min and 30 minutes, particularly between 5 and 15 minutes. In one embodiment, the predefined threshold is about 10 minutes. The predefined threshold may be stored in the memory of the mobile device. The indicated threshold is particularly suitable if the medical values are glucose values as it ensures up to date information without bothering the patient too often about missing glucose values by showing the predefined icon.

The medical application may be configured to receiving the one or more medical values, creating the one or more style information, creating the one or more drawing areas, drawing the one or more texts, and creating the one or more medical value icons, wherein the medical application is further configured to sending a notification comprising the respective created medical value icon for displaying on the screen of the mobile device outside of the medical application by an operating system of the mobile device and optionally for presenting the notification comprising the respective created medical value icon and additional information as a banner alert or pop-up notification in a notification panel on a screen of the mobile device outside of the medical application.

The medical application may also be configured to determining whether the difference between a time of measurement of a medical value and the current time does not exceed the predefined time threshold and/or for retrieving first style information from the memory.

In one embodiment, the mobile device comprises a touch-sensitive display and the notification is not dismissible based on a user swiping the notification in the notification panel off the display.

In one embodiment, the medical value(s) are received wirelessly from a medical sensor, a separate electronic device or from a cloud.

In one embodiment, the first, second of further medical value icon is displayed on the lock screen of the mobile device. In one embodiment, the predefined icon is displayed on the lock screen of the mobile device.

A status bar may be displayed on the screen of the mobile device and the first, second, or further medical value icon, or the predefined icon may be displayed in the status bar. In one embodiment, the status bar is displayed on the home screen, the lock screen and optionally the screens of other applications (running in the foreground). In one embodiment, the status bar may further be defined in that it does not accept text for displaying, or of it does accept text, the text will be displayed for no longer than 30 or 10 seconds.

Icons in the status bar may be classified as notification icons and status icons. Notification icons are linked to individual apps. Status icons are always related to the operating system. Notification icons may particularly be on the left side of the status bar. Status icons may particularly be on the right side of the status bar. Notification icons usually have one or more corresponding notifications in the notification panel. The first, second, further medical value icon, and the predefined icon as described herein particularly are notification icons.

In one embodiment, the medical value icon is displayed on the screen for more than 30 seconds. It may be displayed until it is replaced by a more recent medical value or until it is outdated (when it is determined that the difference between the time of measurement of the displayed medical value and the current time exceeds the predefined time threshold). With an exemplary time threshold of 10 minutes, the medical value icon may thus, e.g., be displayed for up to 10 minutes.

As outlined above, the mobile device specifically may be a mobile communications device. Thus, specifically, the mobile device may be a smart phone. The method steps may be computer implemented or computer assisted by a processor of the mobile device comprising program means for performing the method according to one of the preceding embodiments. The program means may be provided by the medical app installed on the mobile device.

In an aspect of the present invention, a mobile device having at least one display, the mobile device being configured for performing the method according to any of the embodiments as described herein is disclosed.

In a further aspect of the present invention, a computer program including computer executable instructions for performing the method according to any one of the embodiments as described herein is disclosed. In particular, the program is executed on a processor of the mobile device.

In a further aspect, particularly a computer program comprising instructions which, when the program is executed by a mobile device having a display, cause the mobile device to carry out the method according to any of the embodiments as described herein is disclosed.

Thus, generally speaking, disclosed and proposed herein is a computer program including computer-executable instructions for performing the method according to the present invention in one or more of the embodiments enclosed herein when the program is executed on a computer or computer network. Specifically, the computer program may be stored on a computer-readable data carrier. Thus, specifically, one, more than one or even all of method steps as indicated above may be performed by using a computer or a computer network, preferably by using a computer program. The computer specifically may be fully or partially integrated into the mobile device, and the computer programs specifically may be embodied as a software app.

In a further aspect, particularly a computer-readable storage medium, specifically a non-transitory storage medium, comprising instructions which, when executed by a mobile device having a display, cause the mobile device to carry out the method according to any one of the preceding method claims is disclosed.

In the following, the invention will be described in further detail, by way of example, with reference to different embodiments.

The examples are not intended to limit the scope of the present invention, but provide further understanding of the invention.

The figures show:
Fig. 1 shows an embodiment of a mobile device of the present invention displaying a glucose value icon on the lock screen in the status bar.
Fig. 2 shows an embodiment of a mobile device of the present invention displaying the predefined icon on the home screen in the status bar.
Fig. 3 shows an embodiment of the method of the present invention.

### Detailed description of the embodiments

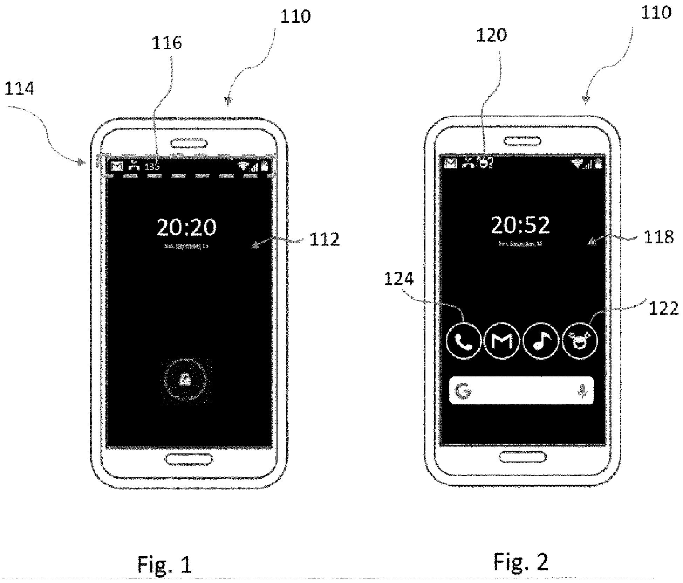

In Figure 1, an exemplary embodiment of a mobile device 110 is shown displaying a medical value icon 116 in the status bar 114 on the lock screen 112. The medical value icon 116 may particularly be a glucose value icon. 135 in this case may stand for a blood glucose level of 135 mg/dl. The status bar in this example comprises other icons than the medical value icon. In the right of the status bar, status icons are displayed such as a battery icon, a signal strength icon and a wifi icon. In the left of the status bar notification icons are displayed, such as the medical value icon 116, a new email icon (e.g., with graphical representation of a small envelope) and a missed call icon (e.g., with graphical representation of a telephone receiver). The status bar may be arranged in the top part of the screen. The status bar only takes a small part of the screen. As an example, the status bar takes up less 1/10, or less than 1/20 of the entire screen displayed. Thereby sufficient parts of the screen are available for displaying other content than notification icons and status icons to the patient. In particular, the medical value icon should only inform the patient in a discrete manner about his current medical state without attracting the attention of others. All icons in the status bar may, e.g., have a size of less than 96x96 pixels. In particular, all icons in the status bar, e.g. have a size of 96x96 or less pixels on a xxxhdpi screen. As another example, all icons in the status bar may be displayed as 24 x 24 dp or less. dp (Density-independent Pixels) is an abstract unit that is based on the physical density of the screen. These units are relative to a 160 dpi screen, so one dp is one pixel on a 160 dpi screen. The notification icons may be displayed on the screen all white. In one embodiment, the medical value icon(s) and/or the predefined icon are thus displayed on the screen all white.

In Figure 2, another exemplary embodiment of a mobile device 110 is shown displaying the predefined icon 120 (also termed unknown current medical status icon) in the status bar on the home screen 118. On the home screen, also a plurality of icons of applications installed on the mobile device may be shown. By tapping such an icon on a touch sensitive display the respective application can be launched so that it will be executed in the foreground. Such icons may thus be termed launcher icons. Launcher icons are displayed bigger on the home screen than notification icons are displayed. Launcher icons, e.g. have at least a 1.5 fold or at least a two-fold, or at least a three-fold size of notification icons. The embodiment of Figure 2 comprises 4 launcher icons displayed on the screen. For example, a launcher icon for a phone app 124 is displayed in the left, followed (from left to right) by a launcher icon for a mail app, followed by a launcher icon for a music app. On the right a launcher icon for the medical app 122 installed on the mobile device is displayed. The medical app may particularly be a diabetes app. In the example of Figure 2, the unknown current medical status icon may be a graphical representation of (parts of) the launcher icon of the medical app and a question mark.

In the examples of Figure 1 and Figure 2, a patient might thus check the lock screen of his mobile device at 20:20 and see the glucose value icon displayed in the status bar. Thereby he is informed about his current medical status, more particularly, this way he may be informed about his current glucose value (135 stands for a blood glucose level of 135 mg/dl). 32 minutes later (at 20:52) he takes a look at the home screen of his mobile device in the example of Figure 2 and sees the unknown current medical status icon. Assuming as an example, that the predefined time threshold is set to 10 min, the patient may thereby be informed that no glucose value has been received on the mobile device that has been measured in the last 10 minutes. This may trigger the patient to check whether his glucose sensor stopped working or whether a connectivity problem might have arisen causing potential problems for the mobile device to receive the current glucose value measured by the glucose sensor.

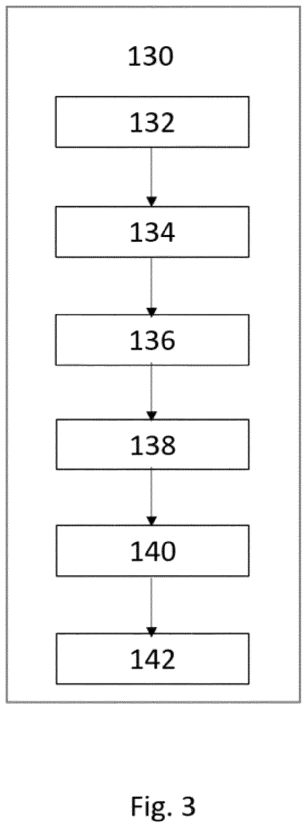

The mobile device 110 is configured, by appropriate programming of a processor comprised in the mobile device, for performing and/or supporting the method according to the present invention. This will be described with reference to an exemplary embodiment shown in a flow chart in Figure 3.

The method 130 comprises the following steps, which may specifically be performed in the given order. Still, a different order may also be possible. It may be possible to perform two or more of the method steps fully or partially simultaneously. It may further be possible to perform one, more than one or even all of the method steps once or repeatedly. The method 130 comprises:
- (denoted with reference number 132) receiving on the mobile device (112) a first medical value and optionally a timestamp associated with the first medical value indicating the time of measurement of the first medical value
- (denoted with reference number 134) converting the first medical value to a first text if the first medical value is not directly received as text
- (denoted with reference number 136) creating first style information for drawing
- (denoted with reference number 138) creating a first drawing area
- (denoted with reference number 140) drawing the first text on the first drawing area using the first style information to create an image of the first medical value
- (denoted with reference number 142) creating a first medical value icon from the image of the first medical value
- (denoted with reference number 144) displaying the first medical value icon (116) on the screen of the mobile device, wherein the screen is a home screen, a lock screen (112) or another screen outside of the medical application (122),
optionally wherein the step of displaying of the first medical value icon, and none, one or more of the steps of converting the first medical value to a first text, creating the first style information, creating the first drawing area, drawing the first text on the first drawing area, and creating the first medical value icon are only conducted if the difference between the time of measurement of the first medical value and the current time does not exceed a predefined time threshold.

The method 130 may comprise additional method steps that are not listed in Figure 3. The method 130 may thus comprise additional steps
- receiving on the mobile device a second medical value after having received the first medical value and optionally a timestamp associated with the second medical value indicating the time of measurement of the second medical value which is after the time of measurement of the first medical value
- converting the second medical value to a second text if the second medical value is not directly received as text
- retrieving the first style information or creating second style information for drawing
- creating a second drawing area
- drawing the second text on the second drawing area using the first style information or the second style information to create an image of the second medical value
- creating a second medical value icon from the image of the second medical value
- replacing the display of the first medical value icon on the screen of the mobile device by displaying the second medical value icon on the screen of the mobile device instead
optionally wherein the step of displaying the second medical value icon, and none, one or more of the steps of converting the second medical value to second text, retrieving the first style information or creating the second style information, creating the second drawing area, drawing the second text on the second drawing area, and creating the second medical value icon are only conducted if the difference between the time of measurement of the second medical value and the current time does not exceed the predefined time threshold
and/or
- determining or re-determining whether the difference between the measurement time of the first, or if a second medical value was received, the measurement time of the second medical value and the current time exceeds the predefined time threshold,
if the difference between the time of measurement of the first, or if a second medical value was received, the time of measurement of the second medical value and the current time exceeds the predefined time threshold replacing the display of the currently displayed medical value icon on the screen of the mobile device by displaying a predefined icon stored in the memory of the mobile phone.

The method may further comprise
- receiving on the medical device a further medical value and a timestamp associated with the further medical value indicating the time of measurement of the further medical value
- determining whether the difference between the time of measurement of the further medical value and the current time does not exceed the predefined time threshold,
- if the time threshold is not exceeded
- converting the further medical value to a further text if the further medical value is not directly received as text
- retrieving the first style information or creating further style information for drawing
- creating a further drawing area
- drawing the further text on the further drawing area using the first style information or the further style information to create an image of the further medical value
- creating a further medical value icon from the image of the further medical value
replacing the display of the currently displayed medical value icon on the screen of the mobile device by displaying the further medical value icon on the screen of the mobile device instead or replacing the display of the currently displayed predefined icon on the screen of the mobile device by displaying the further medical value icon on the screen of the mobile device instead.

The invention is not limited to the particular methodology and protocols, described herein because they may vary. Although any methods and materials similar or equivalent to those described herein can be used in the practice of the present invention, the preferred methods, and materials are described herein. Further, the terminology used herein is for the purpose of describing particular embodiments only and is not intended to limit the scope of the present invention.

## Claims

1. A computer implemented method of displaying a medical value on a screen of a mobile device comprising a medical application, the method comprising the following steps:
- receiving on the mobile device a first medical value and optionally a timestamp associated with the first medical value indicating the time of measurement of the first medical value
- converting the first medical value to a first text if the first medical value is not directly received as text
- creating first style information for drawing
- creating a first drawing area
- drawing the first text on the first drawing area using the first style information to create an image of the first medical value
- creating a first medical value icon from the image of the first medical value
- displaying the first medical value icon on the screen of the mobile device, wherein the screen is a home screen, a lock screen or another screen outside of the medical application,
optionally wherein the step of displaying of the first medical value icon, and none, one or more of the steps of converting the first medical value to a first text, creating the first style information, creating the first drawing area, drawing the first text on the first drawing area, and creating the first medical value icon are only conducted if the difference between the time of measurement of the first medical value and the current time does not exceed a predefined time threshold.

2. The method according to the preceding claim further comprising the following steps
- receiving on the mobile device a second medical value after having received the first medical value and optionally a timestamp associated with the second medical value indicating the time of measurement of the second medical value which is after the time of measurement of the first medical value
- converting the second medical value to a second text if the second medical value is not directly received as text
- retrieving the first style information or creating second style information for drawing
- creating a second drawing area
- drawing the second text on the second drawing area using the first style information or the second style information to create an image of the second medical value
- creating a second medical value icon from the image of the second medical value
- replacing the display of the first medical value icon on the screen of the mobile device by displaying the second medical value icon on the screen of the mobile device instead,
optionally wherein the step of displaying the second medical value icon, and none, one or more of the steps of converting the second medical value to second text, retrieving the first style information or creating the second style information, creating the second drawing area, drawing the second text on the second drawing area, and creating the second medical value icon are only conducted if the difference between the time of measurement of the second medical value and the current time does not exceed the predefined time threshold.

3. The method according to any of the preceding claims further comprising
- determining or re-determining whether the difference between the measurement time of the first, or if a second medical value was received, the measurement time of the second medical value and the current time exceeds the predefined time threshold, and
if the difference between the time of measurement of the first, or if a second medical value was received, the time of measurement of the second medical value and the current time exceeds the predefined time threshold, replacing the display of the currently displayed medical value icon on the screen of the mobile device by displaying a predefined icon stored in the memory of the mobile device.

4. The method according to any of the preceding claims further comprising
- receiving on the medical device a further medical value and a time stamp associated with the further medical value indicating the time of measurement of the further medical value
- determining whether the difference between the time of measurement of the further medical value and the current time does not exceed the predefined time threshold,
- if the time threshold is not exceeded
- converting the further medical value to a further text if the further medical value is not directly received as text
- retrieving the first style information or creating further style information for drawing
- creating a further drawing area
- drawing the further text on the further drawing area using the first style information or the further style information to create an image of the further medical value
- creating a further medical value icon from the image of the further medical value
- replacing the display of the currently displayed medical value icon on the screen of the mobile device by displaying the further medical value icon on the screen of the mobile device instead or replacing the display of the currently displayed predefined icon on the screen of the mobile device by displaying the further medical value icon on the screen of the mobile device instead.

5. The method according to any of the preceding claims, wherein the medical value(s) is/are a glucose value(s), particularly continuous glucose monitoring value(s).

6. The method according to any of the preceding claims, wherein the predefined threshold is between 1 min and 30 minutes, particularly between 5 and 15 minutes.

7. The method according to any of the preceding claims, wherein the mobile device is a smart phone.

8. The method according to any of the preceding claims, wherein the medical application is configured to receiving the one or more medical values, creating the one or more style information, creating the one or more drawing areas, drawing the one or more texts, and creating the one or more medical value icons, wherein the medical application is further configured to sending a notification comprising the respective created medical value icon for displaying on the screen of the mobile device outside of the medical application by an operating system of the mobile device and optionally for presenting the notification comprising the respective created medical value icon and additional information as a banner alert or pop-up notification in a notification panel on a screen of the mobile device outside of the medical application.

9. The method according to the preceding claim, wherein the mobile device comprises a touch-sensitive display and the notification is not dismissible based on a user swiping the notification off the display.

10. The method according to any of the preceding claims, wherein the medical value(s) are received wirelessly from a medical sensor, a separate electronic device or from a cloud.

11. The method according to any of the preceding claims, wherein the first, second of further medical value icon is displayed on the lock screen of the mobile device.

12. The method according to any of the preceding claims, wherein a status bar is displayed on the display of the mobile device and the first or second medical value icon is displayed in the status bar.

13. A mobile device having at least one display, the mobile device being configured for performing the method according to any of the preceding claims.

14. A computer program comprising instructions which, when the program is executed by a mobile device having a display, cause the mobile device to carry out the method according to any one of the preceding method claims.

15. A computer-readable storage medium, specifically a non-transitory storage medium, comprising instructions which, when executed by a mobile device having a display, cause the mobile device to carry out the method according to any one of the preceding method claims.
